Europäisches Patentamt

European Patent Office

Office européen des brevets

(11) Veröffentlichungsnummer: **0 648 839 A1**

## (12) EUROPÄISCHE PATENTANMELDUNG

(21) Anmeldenummer: **94115471.8**

(22) Anmeldetag: **30.09.94**

(51) Int. Cl.6: **C12N 15/52**, C12N 15/82, C12N 9/00, C12N 5/10, C12N 1/21, A01H 5/00, C12Q 1/00

(30) Priorität: **13.10.93 DE 4334791**

(43) Veröffentlichungstag der Anmeldung: **19.04.95 Patentblatt 95/16**

(84) Benannte Vertragsstaaten:
**BE CH DE DK FR GB IT LI NL**

(71) Anmelder: **BAYER AG**

**D-51368 Leverkusen (DE)**

(72) Erfinder: **Kindl, Helmut Prof. Dr.**
**Hans-Meerwein-Strasse**
**D-35043 Marburg (DE)**
Erfinder: **Hain, Rüdiger, Dr.**
**Talstrasse 53a**
**D-40764 Langenfeld (DE)**
Erfinder: **Reif, Hans-Jörg, Prof. Dr.**
**Gottesweg 165**
**D-50939 Köln (DE)**

(54) **Bibenzylsynthase-Gene.**

(57) Die vorliegende Erfindung betrifft neue aus Pflanzen isolierte Gene für Bibenzylsynthase und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Rank Xerox (UK) Business Services
(3.10/3.09/3.3.4)

Die vorliegende Erfindung betrifft neue aus Pflanzen isolierte Gene für Bibenzylsynthase und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Als Bibenzyle werden phenolische Bestandteile, insbesondere 1-(3,5-Dihydroxyphenyl)-2-(3-hydroxyphenyl)-ethan bezeichnet, welche in Orchideen vorkommen und gegenüber Schädlingen, insbesondere Pilzen, Bakterien und Insekten toxisch wirken und somit geeignet sind, diese Schädlinge abzuwehren. Die Fähigkeit der Synthese dieser Substanzen durch die Orchideen wird als wichtiger Abwehrmechanismus angesehen. Von Nutzpflanzen ist die Fähigkeit Bibenzyle zu bilden, bzw. in einem Maße zu erzeugen, welches ihnen eine ausreichende Resistenz gegen Schädlinge verleiht, nicht bekannt.

Die Verwendung von Stilbensynthase-Genen zur Erzeugung von Pflanzen mit einer erhöhten Schädlingresistenz ist bereits aus EP-A 0 309 862 und EP-A 0 464 461 bekannt. In diesen Veröffentlichungen werden speziell Resveratrolsynthase-Gene aus Erdnußpflanzen und Wein beschrieben.

Es wurden nun neue Gene für Bibenzylsynthase ("Bibenzylsynthase-Gene") gefunden, welche in die Erbmasse (das Genom) von Pflanzen eingebaut werden können, die keine Bibenzyle oder nur unzureichend Bibenzyle erzeugen, wodurch eine erhöhte Resistenz dieser Pflanzen gegen Schädlinge hervorgerufen werden kann. Der Begriff Bibenzyle steht für phenolische Bibenzyle, vorzugsweise für 1-(3,5-Dihydroxyphenyl)-2-(3-hydroxyphenyl)-ethan.

Überraschenderweise exprimieren entsprechende transgene Pflanzen Bibenzyle und weisen günstige Schädlingsresistenzen auf, ohne daS für die Pflanzen nachteilige Wirkungen auftreten.

Unter Bibenzylsynthase-Genen soll jede Nukleinsäure (DNA) verstanden werden, die nach ihrer Transkription in RNA und Translation in Protein die Bildung eines Enzyms bewirkt, welches die Eigenschaften einer Bibenzylsynthase (also die Bibenzyl-Bildung bewirkt) besitzt, wobei diese Nukleinsäure aus ihrer natürlichen Umgebung isoliert oder in einen Vektor integriert ist oder in einer prokaryontischen oder eukaryontischen DNA als "fremde" DNA oder als "zusätzliche" DNA enthalten ist.

Unter Bibenzylsynthase-Genen sollen auch solche Bibenzylsynthase-Gene verstanden werden, die an ihrem Anfang und/oder Ende noch DNA-Sequenzen enthalten, die die Funktion der Gene nicht oder nicht wesentlich behindern. Diese auch als "Gen-Einheiten" bezeichneten DNA-Sequenzen entstehen, z.B. durch das Herausschneiden mit Restriktionsenzymen, da keine Schnittstellen für übliche Restriktionsenzyme exakt am Beginn und am Ende des Gens vorliegen. Die Bibenzylsynthase-Gene bzw. die Gen-Einheiten können auch an ihren Enden solche DNA Sequenzen tragen, welche für ihre Handhabung jeweils angepaßt sind (z. B. "Linker").

Die Bibenzylsynthase-Gene (bzw. die Gen-Einheiten) können in der Form vorliegen, wie sie im Genom von Pflanzen enthalten sind ("genomische" Form, einschließlich nicht Bibenzylsynthase kodierender und/oder nicht regulatorisch wirkender Sequenzen (wie Introns)) oder in einer Form, welche der cDNA ("copy" DNA) entspricht, die über mRNA mit Hilfe von Reverse-Transkriptase/Polymerase erhältlich ist (und keine Introns mehr enthält). Die Bibenzylsynthase-Gene können auch in teilweise oder vollständig synthetischer Form vorliegen. Unter synthetischen Genen werden auch solche verstanden, welche durch das neue Zusammenfügen von Teilen entsprechender natürlicher Gene entstehen.

In den erfindungsgemäßen Bibenzylsynthase-Genen (bzw. den Gen-Einheiten) können DNA-Abschnitte durch im wesentlichen gleichwirkende andere DNA-Abschnitte oder DNA's ersetzt sein, solange sie noch die Bibenzylbildung bewirken. Zu den erfindungsgemäßen Genen bzw. Genteilen gehören auch solche, bei denen Variationen von DNA aufgrund der Degenerierung des genetischen Codes vorliegen.

Im vorliegenden Zusammenhang soll unter "fremder" DNA, solche DNA verstanden werden, welche in einem bestimmten prokaryontischen oder eukaryontischen Genom nicht natürlich vorkommt, sondern erst durch Eingriffe durch den Menschen (Transformation) in dieses Genom aufgenommen wird. "Zusätzliche" DNA soll solche DNA sein, welche in dem jeweiligen prokaryontischen oder eukaryontischen Genom zwar natürlich vorkommt, jedoch in zusätzlicher Menge durch Eingriffe durch den Menschen (Transformation) in dieses Genom aufgenommen wird. Die "fremde" DNA oder "zusätzliche" DNA kann je nach Bedarf und Art des vorliegenden Falles in einem oder mehreren Exemplaren eingebaut werden.

Bibenzylsynthase, welche unter Mitwirkung der erfindungsgemäßen Bibenzylsynthase-Gene (bzw. der Gen-Einheiten) in Pflanzen oder Pflanzenzellen gebildet wird, bedeutet jedes Enzym, welches wie Bibenzylsynthase wirkt, also ein oder mehrere Bibenzyle erzeugt, und in Pflanzen deren Resistenz gegenüber Schädlingen erhöht.

Die bevorzugten erfindungsgemäßen Bibenzylsynthase-Gene sind dadurch gekennzeichnet, daß sie mit der im Plasmid pin p8.1.1 enthaltenen cDNA-Sequenz oder ihren Teilen bzw. mit der cDNA-Sequenz gemäß SEQ ID No: 1 oder ihren Teilen hybridisieren und für Bibenzylsynthase codieren.

Erfindungsgemäß bevorzugte Bibenzylsynthase-Gene sind die Bibenzylsynthase-Gene, welche in Orchideen, besonders bevorzugt in Phalaenopsis spp., Bletilla striata oder Epipactis palustris vorkommen und

2

daraus isoliert werden können.

Ganz besonders bevorzugt wird als erfindungsgemäßes Bibenzylsynthase-Gen das Bibenzylsynthase-Gen, dessen Teilsequenz in Form der cDNA auf dem Plasmid p8.1.1 (welches weiter unten näher beschrieben wird) vorliegt, sowie die im wesentlichen gleichwirkenden DNA-Sequenzen. Die cDNA auf dem Plasmid p8.1.1 entspricht dem für Bibenzylsynthase codierenden Strukturgen. Die cDNA auf dem Plasmid p8.1.1 kann somit direkt als Bibenzylsynthase Strukturgen verwendet werden.

Die auf dem Plasmid enthaltene cDNA wurde aus Phalaenopsis isoliert. Sie besteht aus einer ca. 1 600 Basenpaaren langen Sequenz. Eine Teilsequenz von 453 Basenpaaren geht aus dem Sequenzprotokoll SEQ ID No: 1 hervor. Die sich daraus ableitende Proteinsequenz geht aus SEQ ID No: 2 hervor.

Es wurde gefunden, daß die in Orchideen (insbesondere in Phalaenopsis spp., Bletilla striata und Epipactis palustris) vorkommenden Bibenzylsynthase-Gene über weite Bereiche eine DNA-Sequenzhomologie aufweisen. Die erfindungsgemäßen Bibenzylsynthase-Gene können daher auf Grund der Sequenzhomologie mit Hilfe der auf dem Plasmid p8.1.1 enthaltenen cDNA oder ihrer Teile oder den Sequenzinformationen gemäß SEQ ID No:1 in üblicher Weise mit den bekannten Methoden der Molekularbiologie aus Pflanzen in einfacher Weise isoliert werden.

Als Pflanzen, aus denen erfindungsgemäße Bibenzylsynthase-Gene isoliert werden können, kommen praktisch alle Orchideen-Arten, vorzugsweise Phalaenopsis spp., Bletilla striata und Epipactis palustris in Frage.

Wie bereits erwähnt, werden erfindungsgemäß Bibenzylsynthase-Gene bzw. deren codierende Region bevorzugt, die mit der cDNA hybridisieren, welche auf dem Plasmid p8.1.1 liegt. Das Gen bzw. die codierende Region des Gens kann mit Hilfe der cDNA in üblicher Weise erhalten werden. Erfindungsgemäß besonders bevorzugt wird als codierende Region (bzw. Strukturgen) die auf dem Plasmid p8.1.1 enthaltende cDNA-Sequenz oder eine entsprechende DNA-Sequenz, die die Sequenz dieser cDNA-Sequenz enthält.

Der Escherichia coli Stamm E. coli p8.1.1 enthält das Plasmid p8.1.1. Dieser Stamm wurde bei der Deutschen Sammlung von Mikroorganismen (DSM), Mascheroder Weg 1b, D-38124 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt (Hinterlegungsdatum: 27. September 1993). Er erhielt die Hinterlegungsnummer DSM 8580.

Dieser Stamm sowie seine Mutanten sind ebenfalls Teil der vorliegenden Erfindung. Das in diesem Wirt hinterlegte Plasmid p8.1.1 kann in üblicher Weise durch die Vermehrung des Stammes und anschließende Isolierung des Plasmides leicht in den benötigten Mengen gewonnen werden.

Funktionell vollständige Gene, wie die erfindungsgemäßen Bibenzylsynthase-Gene, bestehen aus einem regulatorisch wirkenden Teil (insbesondere Promotor) und dem Strukturgen, welches das Protein Bibenzylsynthase kodiert.

Beide Genteile können unabhängig voneinander verwendet werden. So ist es möglich, dem regulativ wirkenden Teil eine (vom Bibenzylsynthase-Gen abweichende) andere DNA-Sequenz nachzuschalten, welche nach dem Einbau in das Pflanzengenom exprimiert werden soll. Da nur relativ wenige isolierte Promotoren bekannt sind, welche ihre Wirkung in Pflanzen bzw. Pflanzenzellen entfalten können, stellen die Promotoren der Bibenzylsynthase-Gene, welche ebenfalls Bestandteile der vorliegenden Erfindung sind, wertvolle Hilfsmittel bei der Erzeugung transformierter Pflanzen bzw. Pflanzenzellen dar.

Ebenso ist es möglich, den Bibenzylsynthase-Struktur-Genen einen "fremden" regulatorisch wirkenden Teil vorzuschalten. Hierbei können besonders vorteilhaft die Stilbensynthase-Promotoren gemäß EP-A 0 309 862 und EP-A 0 464 461 eingesetzt werden. Dies könnte z.B. vorteilhaft sein, wenn bei bestimmten Pflanzen nur bestimmte (z.B. pflanzeneigene) regulatorisch wirkende Gene ausreichend wirksam werden können. Die Bibenzylsynthase-Struktur-Gene, insbesondere auch die Strukturgene, die der cDNA des Plasmids p8.1.1 entsprechen, bzw. diese Sequenz enthalten stellen somit wertvolle, selbständig einsetzbare Einheiten dar und sind, wie bereits dargelegt, ebenfalls Teil der vorliegenden Erfindung. Die erfindungsgemäßen Bibenzylsynthase-Gene können nach den üblichen Methoden in die regulatorisch wirkenden Teile und die Struktur-Gene getrennt werden. Es ist auch möglich, Teile von verschiedenen natürlich vorkommenden Bibenzylsynthase-Genen zu neuen funktionellen "synthetischen" Genen zu kombinieren. Bevorzugt werden die vollständigen natürlichen erfindungsgemäßen Bibenzylsynthase-Gene (bzw. die Gen-Einheiten) verwendet.

Mit Hilfe der üblichen Methoden ist es möglich, die Bibenzylsynthase-Gene (bzw. die Gen-Einheiten) oder ihre Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. So kann z.B. die proteincodierende DNA mit regulatorischen Sequenzen versehen werden und in Pflanzen eingebaut werden. Die so "modifizierte" rekombinante DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Die Bibenzylsynthase-Gene (bzw. die Gen-Einheiten) und/oder ihre Teile sowie die rekombinante DNA können als "fremde" oder "zusätzliche" DNA in Vektoren (insbesondere Plasmiden, Cosmiden oder Phagen), in transformierten Mikroorganismen (vorzugsweise Bakterien, insbesondere Gram-negativen Bakterien, wie E. coli) sowie in transformierten Pflanzenzellen und Pflanzen bzw. in deren DNA enthalten sein. Solche Vektoren, transformierte Mikroorganismen (die auch diese Vektoren enthalten können) sowie die transformierten Pflanzenzellen und Pflanzen und deren DNA stellen Bestandteile der vorliegenden Erfindung dar.

Wie bereits angedeutet, werden erfindungsgemäß die Bibenzylsynthase-Gene (bzw. die Gen-Einheiten) ein-oder mehrfach (an gleichen oder verschiedenen Stellen des Genoms) in das natürliche pflanzliche Genom eingebaut, wobei verschiedene Gene auch mit einander kombiniert werden können. Bei Pflanzen, welche bereits über die Fähigkeit der Bibenzylsynthase-Synthese verfügen (Orchideen), kann der Einbau eines oder mehrerer erfindungsgemäßer Bibenzylsynthase-Gene zu einem erheblich verbesserten Resistenzverhalten führen. Bei Pflanzen, die keine Bibenzylsynthase-Gene enthalten, wird durch den Einbau solcher Gene ebenfalls eine erhöhte Schädlingsresistenz erreicht. Gegebenenfalls werden nur die erfindungsgemäßen Strukturgene verwendet, wobei ein evtl. aus einer anderen oder derjeweiligen Pflanze isoliertes regulatorisches DNA Element vorgeschaltet wird.

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze, erhöhte Resistenzen aufweisen.

Die vorliegende Erfindung betrifft somit auch ein Verfahren zur Herstellung transgener Pflanzenzellen (einschließlich Protoplasten) und Pflanzen (einschließlich Pflanzenteile und Samen) mit erhöhter Resistenz gegen Schädlinge, welches dadurch gekennzeichnet ist, daß man

(a) ein oder mehrere Bibenzylsynthase-Gene (bzw. Gen-Einheiten) und/oder Teile der Bibenzylsynthase-Gene (bzw. der Gen-Einheiten) und/oder erfindungsgemäße rekombinante DNA, welche DNA-Sequenzen enthalten, die für Bibenzylsynthase codieren, in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transgenen Pflanzen der Elterngeneration oder weiterer daraus gewonnener Generationen die gewünschten Pflanzenteile (einschließlich Samen) gewinnt.

Die Verfahrensschritte (a), (b) und (c) können nach bekannten Verfahren und Methoden in üblicher Weise durchgeführt werden.

Transgene Pflanzenzellen (einschließlich Protoplasten) und Pflanzen (einschließlich Pflanzenteile und Samen), welche ein oder mehrere Bibenzylsynthase-Gene (bzw. Gen-Einheiten) und/oder Teile der Bibenzylsynthase-Gene (bzw. der Gen- Einheiten) als "fremde" oder "zusätzliche" DNA enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der Bibenzylsynthase-Gene (bzw. der Gen-Einheiten) und/oder ihrer Teile und/oder der erfindungsgemäßen rekombinanten DNA und/oder der erfindungsgemäßen rekombinanten Vektoren und/oder der erfindungsgemäßen transformierten Mikroorganismen zur Transformation von Pflanzenzellen (einschließlich Protoplasten) und Pflanzen (einschließlich Pflanzenteilen und Samen), die

(b) Verwendung der erfindungsgemäßen transgenen Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial, die

(c) Verwendung der erfindungsgemäßen Bibenzylsynthase-Gene (bzw. der Gen-Einheiten) und/oder ihrer Teile und/oder der erfindungsgemäßen rekombinanten DNA zur Bekämpfung von Schädlingen sowie die

d) Verwendung der auf dem Plasmid p8.1.1 enthaltenen cDNA oder ihrer Teile sowie der den Sequenzinformationen gemäß Sequenzprotokoll SEQ ID NO:1 entsprechenden DNA Sequenzen zur Isolierung von Bibenzylsynthase-Genen oder deren Teilen aus Pflanzen sowie zur Bestimmung von Bibenzylsynthase-Genen in Pflanzen sowie (allgemein) bei der Erzeugung von transgenen Pflanzenzellen (einschließlich Protoplasten) und Pflanzen (einschließlich Pflanzenteilen und Samen), sowie die Verwendung der durch das Strukturgen von p8.1.1 codierten Proteinsequenz (Bibenzylsynthase) sowie des Proteins gemäß SEQ ID NO:2 bei der Isolierung und dem Nachweis der Bibenzylsynthase-Gene (z.B. durch die übliche Antikörper-Technik). Die durch die erfindungsgemäßen Bibenzylsynthase-Gene (insbe-

4

sondere durch das Struktur-Gen von p8.1.1) codierte Bibenzylsynthase sowie das Protein gemäß SEQ ID NO:2 gehören ebenfalls zur vorliegenden Erfindung.

Eine Anzahl verschiedener Methoden steht zur Verfügung, die Bibenzylsynthase-Gene bzw. die Gen-Einheiten oder ihre Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Das genetische Material, welches für Bibenzylsynthase kodiert, wird zusammen mit regulatorischen DNA-Sequenzen in die T-DNA von geeigneten Ti-Plasmiden eingesetzt (z.B. Zambryski et al.,1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält in Pflanzenprotoplasten (z.B. Hain et al., 1985; Krens et al., 1982; Paszkowski et al., 1984) in Gegenwart von Polykationen oder Calciumsalzen und Polyethylenglykol.

Die DNA-Aufnahme kann auch zusätzlich durch ein elektrisches Feld (Elektroporation) begünstigt werden (z.B. Fromm et al., 1986).

Die DNA kann in bekannter Weise auch über Pflanzenpollen eingeführt werden, indem Pollen oder andere Pflanzenteile mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) werden die erfindungsgemäßen Gene bzw. Geneinheiten in isolierter Form in einen geeigneten intermediaeren E.coli Vektor z.B. pGV700 oder pGV710, (vergl. EP-A 116 718) bzw. vorzugsweise Derivaten davon, die zusätzlich ein Reportergen wie z.B. nptII (Herrera-Estrella et al. 1983) oder hpt (Van den Elzen et al 1986) enthalten, kloniert.

Das so konstruierte Plasmid wird auf Agrobacterium tumefaciens, das z.B. pGV 3850 bzw. Derivate davon enthält (Zambryski et al. 1983) mit üblichen Methoden (z.B. Van Haute et al. 1983) übertragen. Alternativ dazu kann die Bibenzylsynthase-Geneinheit in einem binaeren Vektor, z.B. pCV001 oder pCV002 (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Bibenzylsynthase-Gene bzw. Gen-Einheiten in einer auf Pflanzen transferierbaren Form enthält wird im weiteren zur Pflanzentransformation verwendet.

In einer weiteren bevorzugten Ausführungsform werden die isolierten Bibenzylsynthase-Geneinheiten gegebenenfalls zusammen mit einem anderen Plasmid, das ein Reportergen für Pflanzenzellen, z.B. für Kanamycin-Resistenz (z.B. Herrera-Estrella et al. 1983) oder eine Hygromycin-Resistenz (van den Elzen, 1986) enthält, vorzugsweise pLGV neo 2103 (Hain et al. 1985), pMON 129 (Fraley R.T. et al., Proc. National Acad. Sci. USA 80, 4803 (1983)), pAK 1003, pAK 2004 (Velten J. et al., EMBO Journ. Vol. 3, 2723 (1984)) oder pGSST neo 3 (pGSST3) (EP-A-189 707), in üblicher Weise durch direkten Gentransfer auf Pflanzenprotoplasten übertragen (z.B. Hain et al 1985). Dabei können das bzw. die Plasmide in zirkulärer, vorzugsweise jedoch in linearer Form, vorliegen. Bei der Verwendung eines Plasmids mit Reportergen werden kanamycinresistente Protoplasten dann auf Expression von Bibenzylsynthase überprüft. Im anderen Fall (ohne Reportergen) werden die resultierenden Kalli auf die Expression des oder der Bibenzylsynthase-Gene geprüft (Screening mit üblichen Methoden).

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro (Reiss et al. 1984; Schreier et al. 1985) oder durch die Expression der Nopalinsynthase (nach Aerts et al. 1983) oder Bibenzylsynthase durch Northern-Blot-Analyse und Western Blot-Analyse nachgewiesen. Bibenzylsynthase und die Bibenzyle können auch in bekannter Weise mit Hilfe spezifischer Antikörper in transformierten Pflanzen nachgewiesen werden. Bibenzylsynthase kann auch durch Enzymaktivitätstest nachgewiesen werden.

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche die erfindungsgemäßen Bibenzylsynthase-Gene (bzw. die Gen-Einheiten) enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung der transformierten Pflanzen und Pflanzenzellen verwendet werden können und ist somit ebenfalls Teil der vorliegenden Erfindung.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der Bibenzylsynthase-Gene und ihrer Teile nicht derart beeinträchtigt ist, daß Bibenzylsynthase nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nukleinsäuren erfolgen. Sie können auch im Hinblick auf die Degenerierung des genetischen Codes vorliegen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere das Plasmid p8.1.1 enthalten.

Zu den Pflanzen, welchen durch den Einbau (Transformation) der erfindungsgemäßen Bibenzylsynthase-Gene (bzw. der Gen-Einheiten) Resistenz bzw. eine erhöhte Resistenz gegenüber den Schädlingen verliehen werden kann, gehören praktisch alle Pflanzen außer Orchideen, in welchen Bibenzylsynthase-Gene natürlich vorkommen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbsondere Weizen, Roggen, Gerste, Hafer, Hirse, Reis und Mais), Kartoffel, Leguminosen (wie Hülsenfrüchte und insbesondere Alfalfa, Sojabohnen), Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen wie Rauwolfia und Digitalis. Besonders bevorzugt seien Kartoffel, Tomaten und Leguminosen genannt. Vorzugsweise werden die erfindungsgemäßen Bibenzylsynthase-Gene als "fremde" DNA in den Genom von Pflanzen eingebaut.

Als Schädlinge, gegen welche mit Hilfe der erfindungsgemäßen Bibenzylsynthase-Gene Resistenzen, bzw. erhöhte Resistenzen erzielt werden können, seien tierische Schädlinge, wie Insekten, Milben und Nematoden sowie mikrobielle Schädlinge, wie phytopathogene Pilze, Bakterien und Viren genannt. Besonders hervorgehoben werden mikrobielle Schädlinge, insbesondere phytopathogene Pilze.

Zu den schädlichen Insekten gehören insbesondere Insekten der Ordnungen:
Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere:
Tarsonemus spp., Panonychus spp. und Tetranychus spp.

Zu den schädlichen Nematoden gehören insbesondere:
Pratylenchus spp., Heterodera spp. und Meloidogyne spp.

Zu den mikrobiellen Schädlingen gehören insbesondere die phytopathogenen Pilze:
Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Zu den Viruserkankungen gehören insbesondere Mosaik-, Verzwergungs- und Vergilbungsvirosen.

Beispielhaft aber nicht begrenzend seien einige Erreger von virösen, pilzlichen und bakteriellen Erkrankungen, die unter die oben aufgezählten Oberbegriffe fallen, genannt:
Barley Yellow Dwarf Virus (BYDV), Potato Virus Y (PVY), Cucumber Mosaic Virus (CMV), Watermelon Mosaic Virus (WMV), Tristeza-Virus, Tobacco Mosaic Virus (TMV), Tobacco Necrosis Virus (TNV), Beet necrotic Yellow Vein Virus (BNYVV), Rhizomania-Virus.

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielwseise Pseudocercosporella herpotrichoides. Weiterhin sei Helminthosporium carbonum aufgeführt.

Die vorliegende Erfindung soll anhand der folgenden beispielhaften Ausführungen näher erläutert werden:

## 1. Isolierung des Gens für Bibenzylsynthase aus Orchideen

Pflanzen und Zellkulturen aus Orchideen enthalten die Gene für Bibenzylsynthase, welche die Bildung von Bibenzylsynthase (Größe des Proteins 90.000 D; Reaktion mit spezifischem Antiserum) bewirken. Das Enzym besteht aus einem Homodimer von 43.000 D.

Bei der Isolierung der Bibenzylsynthase-Gene sowie der cDNA Sequenzen insbesondere der auf dem Plasmid p8.1.1 enthaltenen cDNA wurden die bekannten Verfahren und Methoden der Molekularbiologie verwendet, wie sie beispielsweise in folgendem Handbuch detailliert beschrieben werden: Sambrook, J., Fritsch, E.F., Maniatis, T.: Molecular Cloning: A Laboratory Manual; Cold Spring Harbor Laboratory, Second Edition 1989.

Es wird zunächst eine "Gen-Bibliothek" für die jeweiligen Orchideen angelegt: Genomische DNA aus angereicherten Zellkernen (Bedbrook, J., Plant Molecular Biology Newsletter 2, 24, 1981) wird mit dem Restriktions-Enzym NdeII so geschnitten, daß DNA-Fragmente mit einer Durchschnittslänge von etwa 12 000 Nukleotidpaaren entstehen. Diese Fragmente werden in die BamHI-Stelle von Lambda-Phage EMBL4 kloniert (Frischauf et al., J. Mol. Biol. 170, 827-842, 1983), und die Phagen werden in E. coli vermehrt. Die Gesamtheit der Phagen-Population enthält, kloniert in Teilstücken, die gesamte genomische DNA der Orchideenzellen, und damit auch die Gene für Bibenzylsynthasen (Multigenfamilie).

Die Gene für Bibenzylsynthase, ihre mRNA und die Bibenzylsynthasen-cDNA enthalten jeweils gleiche Nucleinsäuresequenzen, da sie voneinander abgeleitet werden können (Gen→mRNA→cDNA). Dies bedeutet, daß die Gene für Bibenzylsynthase durch spezifische Hybridisierung mit der jeweiligen Bibenzylsynthase-cDNA bzw. mit spezifischen Oligonukleotiden identifizierbar sind. Gemäß diesem Verfahren werden genomische Phagen-Klone für Bibenzylsynthasen identifiziert und in Tabak übertragen mit dem Ergebnis, daß Bibenzyl (1-(3,5-Dihydroxyphenyl)-2-(3-hydroxyphenyl)-ethan) in den heterologen Pflanzen erzeugt werden. Die transgenen Pflanzen weisen eine erhöhte Resistenz gegen Pflanzenpathogene auf. Die Phagen mit den Genen werden durch Hybridisierung identifiziert, dann isoliert und vermehrt. Die in diesen Phagen klonierte genomische DNA aus Orchideen wird weiter durch Analyse mit verschiedenen Restriktionsenzymen kartiert, und die Position der Bibenzylsynthase-Gene wird durch weitere Hybridisierungs-Experimente mit cDNA-Sequenzen bzw. synthetischen Oligonukleotiden festgelegt. Schließlich werden die Gen-Einheiten durch Verdau mit Restriktionsenzymen aus dem Phagen herausgeschnitten, im entsprechend geschnittenen Plasmid-Vektor pUC18 kloniert (Fa. Gibco-BRL GmbH, Eggenstein, Bundesrepublik Deutschland), und als rekombinante Plasmide vermehrt.

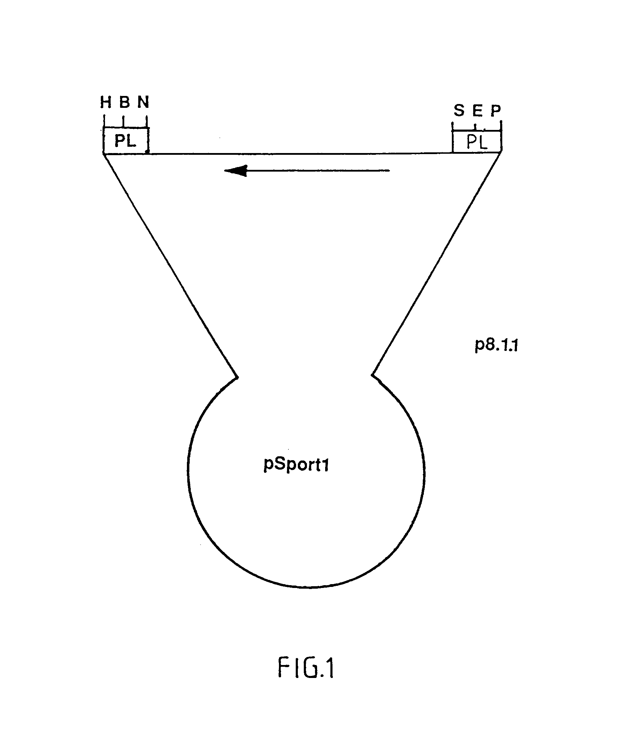

## 2. Beschreibung des Plasmids p8.1.1 (vgl. Fig. 1)

Das Plasmid besteht aus zwei Komponenten:

(i) cDNA von Bibenzylsynthase: Die cDNA, welche in das Plasmid pSport1 (Gibco/BRL) eingesetzt wurde, ist 1,6 kb lang und kann mit MluI aus dem Plasmid p8.1.1 herausgeschnitten werden.

(ii) Vektor-Plasmid: Die cDNA ist im Vektor pSport1 (Gibco/BRL, Eggenstein, Bundesrepublik Deutschland) kloniert. Die Größe des Vektors ist 4109 Nukleotidpaare. Er trägt das Gen für Ampicillin-Resistenz, d.h. E. coli-Zellen mit diesem Plasmid wachsen in Nährmedien, die das Antibiotikum Ampicillin enthalten. Ori: Bezeichnung für Sequenzen, die für die Vermehrung des Plasmids in E. coli notwendig sind.

Das Plasmid p8.1.1 trägt ein Gen für Ampicillin-Resistenz und enthält als Bibenzylsynthase-cDNA das oben beschriebene MluI-Fragment mit etwa 1,6 kb. Es kann in E. coli Zellen, welche p8.1.1 enthalten (E. coli p8.1.1), in üblicher Weise vermehrt werden.

Bevorzugtes Nährmedium für E. coli-Zellen (z.B. JA221, Nakamura, K., Inouye, M., EMBO J. 1, 771-775, 1982) welche p8.1.1 enthalten (E. coli pin 5-49):

| Bacto-Pepton* | 10 g |
|---|---|
| Hefeextract | 5 g |
| NaCl | 5 g |
| Agar | 20 g |
| $H_2O$ | 1 l |
| pH 7,5 | |
| Fermentation: 37°C, aerob | |

(* Bacto ist ein Warenzeichen der Fa. DIFCO Lab. Detroit, USA).

## 3. Transformation von Tabak

a) Kultur von Tabaksprossen und Isolierung von Tabakprotoplasten:

Nicotiana tabacum (Petit Havana SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x $10^5$/ml verdünnt. Die Protoplastenkonzentration wird in einer Zählkammer bestimmt.

b) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von 1-2 x $10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin) 2 Tage im Dunkeln und ein bis zwei Tage lang unter Schwachlicht (500 lux) bei 26°C inkubiert. Sobald die ersten Teilungen der Protoplasten auftreten, werden 30 μl einer Agrobakteriumsuspension in minimal A (Am) Medium (Dichte ca. $10^9$ Agrobakterien/ml) zu 3 ml regenerierenden Protoplasten gegeben. Die Kokulturdauer beträgt 3-4 Tage bei 20°C im Dunkeln. Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x $10^4$/ml in K3 Medium (0,3 m Saccharose) mit 1 mg/l NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose "bead type culture" (Shillito et al. 1983) gestartet. Kanamycinresistente Kolonien können 3-4 Wochen nach Beginn der Selektion vom Hintergrund zurückgebliebener Kolonien unterschieden werden.

c) Direkte Transformation von Tabakprotoplasten mit DNA. Calciumnitrat-PEG Transformation.

In einer Petrischale werden ca. $10^6$ Protoplasten in 180 $\mu$l K3 Medium mit 20 $\mu$l wäßriger DNA Lösung welche 0,5 $\mu$g/$\mu$l Plasmid, welches das genomische Bibenzylsynthasegen oder ein chimäres Gen bestehend aus dem Stilbensynthase-Promotor gemäß EP-A 0 309 862 oder EP-A 0 464 461 und der cDNA für Bibenzylsynthase aus p8.1.1 sowie einer Polyadenylierungssequenz, vorzugsweise aus den Stilbensynthase-Gene der EP-A 0 309 862 oder EP-A 0 464 461 trägt und 0,5 $\mu$g/$\mu$l pLGV neo 2103 (Hain et al. 1985) enthält, vorsichtig gemischt. Anschließend werden 200 $\mu$l Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleinen Menge K3 Medium aufgenommen und wie im nächsten Abschnitt beschrieben kultiviert. Alternativ können die Protoplasten nach Hain et al. 1985 tranformiert werden.

Die Transformation kann auch ohne den Zusatz der 0,5 $\mu$g/$\mu$l pLGV neo 2103 durchgeführt werden. Da in diesem Fall kein Reportergen eingesetzt wird, werden die resultierenden Kalli auf das Vorhandensein der Bibenzylsynthase-Gen-Einheit bzw. des chimären Gens mit Hilfe einer Dot-Blot-Hybridisierung überprüft. Als Hybridisierung-Probe ist die cDNA Sequenz aus p8.1.1 verwendbar. Selbstverständlich können auch andere Nachweismethoden, wie Test mit Antikörpern oder Feststellung einer Pilz-Resistenz eingesetzt werden.

d) Kultur der mit DNA inkubierten Protoplasten und Selektion Kanamycin resistenter Kalli:

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. c) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

Schema der Selektion kanamycinresistenter Tabakkolonien nach DNA Transformation:

```
0,4 M  0,3 M  0,25 M  0,20 M  0,15M  0,10 M  Saccha
                                                rose im
                                                Flüssig
                                                medium

A   E S                                    K
_____

        1       2       3       4       5       6  Wochen
                                                   nach
DNA

Aufnahme
        (K3 Medium 1 mg NAA, 0,2 mg Kinetin)
A = DNA Aufnahme
E = Einbettung in Agarose
S = Selektion mit Kanamycin (100 mg/l Kanamycinsulfat)
K = Kanamycinresistente Kolonien können vom Hintergrund
    eindeutig unterschieden werden
```

e) Regeneration kanamycinrestistenter Pflanzen:

Sobald die kanamycinrestistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamydinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

f) Tranformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien (ca. $10^9$/ml) (vgl. b) in Am-Medium, siehe unten) für ca. 5 Minuten inkubiert. Die infizierten Blattstücke werden auf MS-Medium (siehe unten) ohne Hormone für 3-4 Tage bei ca. 24°C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 $\mu$g/ml Cefotaxim und 100 $\mu$g/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Bibenzylsynthase Aktivität auf Transformation getestet weren. Auf diese Weise werden 1-10 % transformierte Sproße erhalten. Antikörper gegen Bibenzylsynthase oder das Protein gemäß SEQ ID NO:2 können auf übliche Weise erhalten und ebenfalls zum Nachweis der erfolgten Transformation verwendet werden.

Biochemische Nachweismethode der Transformation

Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 $\mu$l des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15 % Essigsäure, 80 % $H_2O$, pH 1,8) getränkt und bei 400 V für 45 Minuten elektrophoretisiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen.

Das getrocknete Papier wird unter langwelligem UV-Licht betrachtet und fotografiert. Arginin und Argininderivate werden mit dem Reagenz gelb fluoreszierend gefärbt.

Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgewiesen. 50 mg Pflanzengewebe werden in 50 $\mu$l Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 $\mu$l des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM $MgCl_2$, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 $\mu$g/ml) und 20-200 $\mu$Ci $^{32}P$ ATP (Amersham) enthält, überschichtet. Das Sandwichgel wird 30 Min. bei Zimmertempreratur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak).

## 4. Transformation von Solanum tuberosum (Kartoffel)

Die Transformation wird genau nach dem in der EP-A-0 242 246, Seiten 14 bis 15 angegebenen Weise vorgenommen, wobei die Agrobakterien Ti-Plasmide enthalten, die Bibenzylsynthase-Gene tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen (Tabak) wird die Anwesenheit der Bibenzylsynthase-Gene durch Southern Blot Analyse bestätigt. Die Expression der Bibenzylsynthase-Gene wird durch Northern Blot Analyse, Bibenzylsynthase und Bibenzyl mit Hilfe von spezifischen Antikörpern nachgewiesen. Transformierte und nicht-transformierte Pflanzen (zum Vergleich) werden mit einer Sporensuspension von Botrytis cinera besprüht und nach 1 Woche der Pilzbefall bonitiert. Die transformierten Pflanzen zeigten (gegenüber den nicht transformierten Vergleichspflanzen) eine erhöhte Resistenz gegen Pilzbefall.

Hybridisierung mit der cDNA-Sequenz von Plasmid p.8.1.1 bzw. der cDNA-Sequenz gemäß SEQ ID No: 1

Wie oben ausgeführt sind die bevorzugten erfindungsgemäßen Bibenzylsynthase-Gene dadurch gekennzeichnet, daß sie mit der im Plasmid p8.1.1 enthaltenen cDNA-Sequenz oder ihren Teilen bzw. mit der cDNA-Sequenz gemäß SEQ ID No: 1 oder ihren Teilen hybridisieren und für Bibenzylsynthase codieren. Die Hybridisierung kann allgemein auch für die Bestimmung und Isolierung von Bibenzylsynthase-Genen z.B. in Pflanzen oder Pflanzenteilen eingesetzt werden.

Vorzugsweise Phagen-Klone die Bibenzylsynthase-Gene enthalten, können durch Hybridisierung mit p8.1.1 (bzw. SEQ ID No: 1) unter niedrig stringenten Bedingungen identifiziert werden. Es wird eine Subpopulation von Klonen erhalten, die nachfolgend als Bibenzylsynthase-Gene-Klone identifiziert werden können, z.B. durch direkten Gen-Transfer in Pflanzen (Hain et al, 1985 und 1990) und anschließende Analyse des transgenen Pflanzengewebes auf Bibenzylsynthase oder das Protein der SEQ ID No:2 (mit Antikörpern) enzymatische Bibenzylsynthase-Aktivität oder auf Bibenzyl.

Beispielhaft wurden Bibenzylsnthase-Gen-Klone mit Hilfe des cDNA-Klones p8.1.1 (bzw. SEQ ID No: 1) als Sonde identifiziert, wobei Standard Hybridisierungsbedingungen eingesetzt wurden. Die Hybridisierung erfolgte 12 Stunden bei 68°C in Standard-Puffer enthaltend 2 SSC. Gewaschen wurde bei 74°C in 2 SSC und 0,1 % SDS (2 mal 30 Min.) mit einer nachfolgenden Waschung in 0,2 SSC und 0,1 % SDC (10 Min.). Die Phagen-Klon-DNA wurde mit einem pflanzenselektiven Marker (Kanamycin-Resistenz) in Tabak-Protoplasten co-übertragen und die Bibenzylsynthase in Tabak nachgewiesen. Ein entsprechendes Ergebnis wurde durch Expression der cDNA aus dem Plasmid p8.1.1 unter geeigneten Promotoren (z.B. Stilbensynthasepromotor gemäß EP-A 0 309 862 oder EP-A 0 464 461) erhalten.

Im folgenden werden einige der bei der Transformation von Pflanzen bzw. Pflanzenzellen eingesetzte Medien beschrieben:

Am-Medium

3,5 g $K_2HPO_4$
1,5 g $KH_2PO_4$
0,5 g $Na_3$ Citrat
0,1 g $MgSO_4$ x $7H_2O$
1 g $(NH_4)_2SO_4$
2 g Glukose
    ad 1 l

Medium für sterile Sproßkultur von Tabak

Macro-elemente 1/2 der Konzentration der MS Salze
Micro-elemente 1/2 der Konzentration der MS Salze

| Fe-EDTA | Murashige und Skoog (MS) | |
|---|---|---|
| Myo-Inosit | | 100 mg/l |
| Sucrose | | 10 mg/l |
| Agar | | 8 g/l |
| Vitamine | Ca-panthotenat | 1 mg/l |
| | Biotin | 10 mg/l |
| | Nicotinsäure | 1 mg/l |
| | Pyridoxin | 1 mg/l |
| | Thiamin | 1 mg/l |
| pH 5,7 vor dem Autoklavieren | | |

K3-Medium

Zur Kultur von Nicotiana tabacum petit Havana SR1, Nicotiana tabacum Wisconsin 38, und Nicotiana plumbaginifolia Protoplasten (Nagy und Maliga, 1976)

| Macro-elemente | $NH_4NO_3$ | 250 mg/l |
| | $KNO_3$ | 2500 mg/l |
| | $CaCl_2 \cdot 2H_2O$ | 900 mg/l |
| | $MgSO_4.7H_2O$ | 250 mg/l |
| | $NaH_2PO_4.1H_2O$ | 150 mg/l |
| | $(NH_4)_2SO_4$ | 134 mg/l |
| | $CaHPO_4.1H_2O$ | 50 mg/l |
| Micro-elemente | $H_3BO_3$ | 3 mg/l |
| | $MnSO_4.1H_2O$ | 10 mg/l |
| | $ZnSO_4.4H_2O$ | 2 mg/l |
| | KI | 0,75 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Sucrose | | 137 g/l (= 0,4 M) |
| Xylose | | 250 mg/l |
| Vitamine | Nicotinsäure | 1 mg/l |
| | Pyridoxin | 1 mg/l |
| | Thiamin | 10 mg/l |
| Hormone | NAA | 1,0 mg/l |
| | Kinetin | 0,2 mg/l |
| pH 5,6 Filter sterilisieren | | |

Linsmaier und Skoog Medium (Linsmaier und Skoog 1965)

Zur Kultur von regenerierenden Protoplasten und für Gewebekultur von Tabaktumoren und Kallus. Linsemaier und Skoog (LS) Medium ist Murashige und Skoog Medium (Murashige und Skoog, 1962) mit den folgenden Modifikationen:
- Thiamin wird in höherer Konzentration eingewogen 0,4 mg/l anstatt 0,1 mg/l;
- Glycin, Pyridoxin und Nicotinsäure fehlen.

| Macro-elemente | $NH_4NO_3$ | 1650 mg/l |
| | $KNO_3$ | 1900 mg/l |
| | $CaCl_2.2H_2O$ | 440 mg/l |
| | $MgSO_4.7H_2O$ | 370 mg/l |
| | $KH_2PO_4$ | 170 mg/l |
| Micro-elemente | $H_3BO_3$ | 6,2 mg/l |
| | $MnSO_4.1H_2O$ | 22,3 mg/l |
| | $ZnSO_4.4H_2O$ | 8,6 mg/l |
| | KI | 0,83 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Saccharose | | 30 g/l |
| Agar | | 8 g/l |
| Vitamine | Thiamin | 0,4 mg/l |
| Hormone: | NAA | 1 mg/l |
| | Kinetin | 0,2 mg/l |
| pH 5,7 vor dem Autoklavieren | | |

### 5. Induktion und Reinigung der Bibenzylsynthase aus Bletilla striata

#### 5.1 Material und Methoden

#### 5.1.1 Pflanzenmaterial

Es wurden Knollen von Bletilla striata verwendet.

#### 5.1.2 Festagarkulturen von Botrytis cinerea und Rhizoctonia crocorum

Die Kulturen wurden auf Kartoffel-Dextrose-Agarplatten (PDA-Platten, DIFCO Detroit, Mich. USA) angezogen.

#### 5.1.3 Induktion der Bibenzylsynthase

Die Knollen wurden geschält, mit einem Skalpell in etwa 2 mm dicke Scheiben geschnitten und bei 100 % Luftfeuchtigkeit und 20°C gelagert.

Zur zusätzlichen Behandlung mit Pilz wurde auf eine 1 Monate alte Agarplatte von Botrytis bzw. Rhizoctonia 10 ml Leitungswasser aufgebracht und Mycel (Rhizoctonia) bzw. Mycel + Konidien (Botrytis) mit einem Spatel in Suspension gebracht.

In die so erhaltene Suspension wurden die Orchideen-Scheiben für 1 Minute getaucht und dann wie oben beschrieben gelagert. Die induzierten Orchideen-Scheiben wurden nach dem Einfriereen in flüssigem Stickstoff bei -80°C gelagert.

#### 5.1.4 Synthese von CoA-Estern substituierter Zimt- und Phenylpropionsäuren

Die CoA-Ester wurden nach der von Stöckigt und Zenk (1975), Z. Naturforsch. 30C, Seite 352-358 beschriebenen Methode hergestellt. Die Säure wurde in den Hydroxysuccinimidester überführt und dann in den CoA-Ester umgeestert. Die Reinigung der CoA-Ester erfolgte durch Papierchromatographie.

5.1.5 Aktivitätstest der Bibenzylsynthase

Der routinemäßige Test wurde mit Dihydro-m-coumaroyl-CoA als Substrat durchgeführt.

Testansatz:    5 $\mu$l [2-$^{14}$C]Malonyl-CoA (1,85 TBq/mol; 370 Bq/$\mu$l)

40 $\mu$l Enzymlösung

5 $\mu$l Substratlösung (1 mM)

Inkubation:    30 min bei 30 °C Wasserbad

Teststop:    500 $\mu$l bidest.

10 $\mu$l Naringenin (1 mg/ml Ethylacetat)

Der Testansatz wurde zweimal mit je 1 ml Ethylacetat 20 sec ausgeschüttelt. Die vereinten organischen Phasen wurden in einer Vakuumzentrifuge eingeengt, zweimal mit je 20 $\mu$l Ethylacetat aufgenommen und als 2,5 cm breite Bahn auf einer Kieselgel-Dünnschichtplatte (mit Fluoreszenzindikator 254 nm) aufgetragen. Laufmittel: EtOAc:Toluol:Aceton (50:35:15 Vol-Teile) Das Radioaktivitätsprofil der Platte wurde mit einem Dünnschichtscanner (LB 2723, Fa. Berthold) aufgenommen. Die Produktzonen wurden ausgekratzt, mit Szintillationsflüssigkeit (11 eco, Fa. Roth) versetzt und ausgezählt.

5.1.6 Proteinbestimmung

Die Proteinkonzentration wurde mit dem Mikro Assay (Fa. Bio-Rad) nach einer modifizierten Methode von Bradford (1976) Anal. Biochem. 72, 248 durchgeführt. Als Eichprotein diente BSA.

5.1.7 Elektrophoretische Methoden

5.1.7.1 Probenvorbereitung

Protein wurde mit dem vierfachen Volumen Aceton bei -20 °C innerhalb von 30 min gefällt, mit 80 % (v/v) Aceton und 80 % (v/v) Ethanol gewaschen, in einer Vakuumzentrifuge getrocknet und in Extraktionsmedium durch zweiminütiges Kochen gelöst.

5.1.7.2 SDS-Page

Die Proteinauftrennung erfolgte nach der Methode von Laemmli (1970) Nature 227, Seite 680-685 in einer Minigelapparatur der Fa. Biometra.

5.1.7.3 Proteinfärbung

Coomassie-Anfärbung:
Das Gel wurde 20 min in Färbelösung geschwenkt. Der Hintergrund wurde mit Entfärbelösung entfärbt.

Färbelösung:    Coomassie Brillant Blue R 250 0,25 % (w/v) Methanol 50 % (v/v) Eisessig 5 % (v/v)

Entfärbelösung:    Isopropanol 40 % (v/v) Eisessig 10 % (v/v)

Silberanfärbung:    Zur Anwendung kam eine Methode von Wray et al. (Wray, W., Boulikas, T., Wray, V.P., Hancock, R. (1981) Anal. Biochem. 118, 197-203).

5.1.8 Chromatofokussierung

Die Bestimmung des isoelektrischen Punktes erfolgte durch Chromatofokussierung an PBE 94. Die CF wurde mit einer angereicherten Bibenzylsynthase-Präparation durchgeführt.

5.1.8.1 Durchführung

Chromatographiematerial:    PBE 94 (Fa. Pharmacia)

Säulendimension:    1,0 x 22 cm; 17 ml

Äquilibriert:    25 mM Histidin (HCl, pH 6,2, 2 mM 2-Mercaptoethanol

Elution mit:    Polybuffer 74-HCl (1:8 mit bidest. verdünnt), pH 4,0, 2 mM Mercaptoethanol (Startpuffer)

Flußrate: 22 ml/h
Fraktionsvolumen: 3 ml

Die Bibenzylsynthase wurde durch Chromatographie an HAP und Sephacryl S-200 HR (s. Reinigung) angereichert und in den Startpuffer der CF überführt.

Auf die Chromatofokussierungssäule wurde der Aktivitätspeak der Chromatographie an S-200 (48 ml; Gesamtaktivität = 5,4 pkat in Startpuffer) mit einer Flußrate von 12 ml/h aufgetragen.

1.8.2 Ergebnis

| Fraktion | pH |
|---|---|
| 1 | 6.2 |
| 5 | 6.2 |
| 10 | 6.1 |
| 15 | 5.8 |
| 20 | 5.6 |
| 25 | 5.3 |
| 30 | 5.0 |
| 35 | 4.8 |
| 40 | 4.5 |
| 45 | 4.2 |
| 50 | 4.0 |

| Fraktion | Aktivität (pkat/ml) |
|---|---|
| 34 | 0.01 |
| 35 | 0.02 |
| 36 | 0.08 |
| 37 | 0.12 |
| 38 | 0.09 |
| 39 | 0.13 |
| 40 | 0.10 |
| 41 | 0.04 |
| 42 | 0.02 |

5.2 Reinigung der Bibenzylsynthase (aus Bletilla striata)

Puffer

P1: 25 mM Kaliumphosphat, pH 8,0, 2 mM 2-Mercaptoethanol
P2: 250 mM Kaliumphosphat, pH 8.0, 2 mM 2-Mercaptoethanol
P3: 10 mM Kaliumphosphat, pH 6,4, 2 mM 2-Mercaptoethanol
p4: 25 mM Tris/HCl, pH 8,0, 2 mM 2-Mercaptoethanol

Säulenchromatographische Methoden

Hydroxylapatit:

Chromatographiematerial: Bio-Gel HTP (Fa. Bio-Rad)
Säulendimension: 2,0 x 20 cm; 63 ml
Äquilibriert mit: P1
Linearer Gradient: 150 ml P1 + 150 ml P2
Flußrate der Elution: 25 ml/h
Fraktionsvolumen: 6 ml

Molekularsiebchromatographie

| | |
|---|---|
| Chromatographiematerial: | Sephacryl S-200 HR (Fa. Pharmacia) |
| Säulendimension: | 2,3 x 105 cm; 436 ml |
| Äquilibriert mit: | P3 |
| Flußrate der Elution: | 30 ml/h |
| Fraktionsvolumen: | 4 ml |

Affinitätschromatographie:

| | |
|---|---|
| Chromatographiematerial: | Reactive Red 120-Agarose, Type 3000-Cl (Fa. Sigma) |
| Säulendimension: | 1,1 x 6,0 cm; 5,7 ml |
| Äquilibriert mit: | P3 |
| Elution mit: | P2 |
| Flußrate: | 10 ml/h |

Anionenaustauschchromatographie (FPLC):

| | |
|---|---|
| Säule: | Mono Q HR 5/5 (Fa. Pharmacia) |
| Äquilibriert mit: | P4 |
| Linearer Gradient: | 0-250 mM NaCl |
| Flußrate der Elution: | 1 ml/min |
| Fraktionsvolumen: | 1 ml |

80 g induzierte Orchideenknollen wurden in einer Reibschale unter flüssigem Stickstoff zu einem feinen Pulver zermahlen, mit 1000 ml P1 und 20 g Polyclar AT (Fa. Serva) versetzt, 5 x 15 sec mit einem Ultraturrax gemixt und 30 min bei 12.000 g zentrifugiert.

Die Viskosität des Rohextraktes verhinderte das direkte Auftragen auf eine Chromatographie-Säule. Die Chromatographie an Hydroxylapatit wurde daher teilweise im batch-Verfahren durchgeführt. Der Rohextrakt wurde mit einer Suspension von 20 g HAP in 100 ml P1 versetzt und 3 h bei 200 rpm geschüttelt.

Zur Abtrennung eines Großteils der Zucker und Schleimstoffe wurde das HAP niedertourig abzentrifugiert (1500 g), dreimal mit je 500 ml P1 gewaschen und in eine Säule überführt.

Die Elution erfolgte mit einem linearem Phosphat-Gradatienten von 25-250 mM. Die Bibenzylsynthase eluiert bei 160-180 mM Phosphat.

Die Fraktionen höchster spezifischer Aktivität (48 ml) wurden in einem Dialyseschlauch mit Aquacide (Fa. Calbiochem) auf 15 ml eingeengt und auf das Molekularsieb aufgetragen.

Die aktivsten Fraktionen (12 ml) der Chromatographie an Sephacryl S-200 HR wurden vereint auf eine mit Reactive Red 120-Agarose gefüllte Säule aufgetragen (Flußrate 6 ml/h).

Nicht gebundene Proteine wurden mit 50 ml P3 entfernt (Flußrate 80 ml/h). Die Elution erfolgte mit P2.

Der Aktivitätspeak von 4,5 ml wurde über PD 10 (Fa. Pharmacia) in P4 umgepuffert und mit einer Flußrate von 0,5 ml/h auf Mono Q aufgetragen.

Die Elution erfolgte mit einem linearem NaCl-Gradienten von 0-250 mM in P4. Die Bibenzylsynthase eluierte bei 195-230 mM NaCl. (Fraktionen 10-18).

Zum Thema "Bibenzyle kann folgende Literatur angegeben werden:

Gäumann, E. and Kern, H. (1959) Phytopathol. Z. 36,1. Arditti, J. (1979) Adv. Bot. Res. 7, 421.
Stoessl, A. (1982) in Phytoalexins (Bailey, J.A. and Mansfield, J.W., eds.) p. 133, Blackie, Glasgow.
Majumder, P., Laha, S. and Datta, N. (1982) Phytochem. 21, 478.
Majumder, P.L. and Sen, R.C. (1987) Phytochem. 26, 2121.

Reinigung der Bibenzylsynthase aus Bletilla striata

| Fraktion | Gesamt-Protein (mg) | Gesamt-Aktivität (pkat) | spezif. Aktivität (pkat/mg) | Ausbeute an Aktivität (%) | Faktor der Anreicherung |
|---|---|---|---|---|---|
| Rohextrakt | 408,40 | 8,32 | 0,02 | 100 | 1 |
| Hydroxylapatit-Eluat | 17,18 | 6,86 | 0,40 | 82 | 20 |
| Sephacryl S200 HR-Eluat | 2,24 | 5,74 | 2,56 | 69 | 128 |
| Red Agarose-Eluat | 1,16 | 3,58 | 3,09 | 43 | 155 |
| Mono Q-Eluat | 0,11 | 0,50 | 4,55 | 6 | 228 |

Majumder, P.L. and Chatterjee, S. (1989) Phytochem. 28, 1986.

Zur Transformation von Pflanzen bzw. Pflanzenzellen kann die folgende Literatur angeführt weren:

Aerts M, Jacobs M, Hernalsteens JP, Van Montagu M, Schell J (1983) Induction and in vitro culture of Arabidopsis thaliana crown gall tumours. Plant Sci Lett. 17: 43-50

Fromm ME, Taylor LP, Walbot V (1986) Stable transformation of maize after gene transfer by electroporation. Nature 319: 791-793

Hain, R., Stabel, P., Czernilofsky, A.Pp., Steinbiß, H.H., Herrera-Estrella, L., Schell, J. (1985) Uptake, integration, expression and genetic transmission of a selectable chimeric gene by plant protoplasts. Molec Gen Genet 199: 161-168

Herrera-Estrella L., De Block M., Messens E., Hernalsteens JP., van Montagu M., Schell J. (1983) EMBO J. 2: 987-995.

Horsch RB, Fry JE, Hoffmann NL, Eichholtz D, Rogers SG, Fraley RT (1985) A simple and general method for transferring genes into plants. Science 277: 1229-1231

Krens FH, Molendijk L, Wullems GJ, Schilperoort RA (1982) in vitro transformation of plant protoplasts with Ti-plasmid DNA. Nature 296: 72-74

Koncz C, Schell J (1986) The promotor of $T_L$-DNA gene 5 controls the tissue-specific expression of chimaeric genes carried by a novel type of Agrobacterium binary vector. Mol. Gen. Genet. (1986) 204: 338-396

Linsmaier DM, Skoog F (1965) Organic growth factor requirements of tobacco tissue cultures. Physiol Plant 18: 100-127

Marton L, Wullems GJ, Molendijk L, Schilperoort PR (1979) In vitro transformation of cultured cells from Nicotiana tabacum by Agrobacterium tumefaciens. Nature 277: 1229-131

Murashige, T. and Skoog F. (1962) A revised medium for rapid growth and bioassay with tobacco tissue culture. Physiol. Plant. 15, 47

Nagy JI, Maliga P (1976) Callus induction and plant regeneration from mesophyll protoplasts of Nicotiana sylvestris. Z Pflanzenphysiol 78: 453-455

Otten LABM, Schilperoort RA (1978) A rapid microscale method for the detection of Lysopin and Nopalin dehydrogenase activities. Biochim biophys acta 527: 497-500

Paszkowski J, Shillito RD, Saul M, Mandak V, Hohn T, Hohn B, Potrykus I (1984) Direct gene transfer to plants. EMBO J 3: 2717-2722

Shillito RD, Paszkowski J. Potrykus I (1983) Agarose plating and Bead type culture technique enable and stimulate development of protoplast-derived colonies in an number of plant species. Pl Cell Rep 2: 244-247 Van den Elzen PJM, Townsend J, Lee KY, Bedbrook JR (1985) Achimaeric resistance gene as a selectable marker in plant cells. Plant Mol. Biol. 5, 299-302.

Van Haute E, Joos H, Maes M, Warren G, Van Montagu M, Schell J (1983) Intergenic transfer and exchange recombination of restriction fragments cloned in pBR322: a novel strategy for the reversed genetics of Ti plasmids of /Agrobacterium tumefaciens. EMBO J 2: 411-418

Velten J, Velten L, Hain R, Schell J (1984) Isolation of a dual plant promotor fragment from the Ti Plasmid of Agrobacterium tumefaciens. EMBO J 12: 2723-2730

Wullems GJ, Molendijk L, Ooms G, Schilperoort RA (1981) Differential expression of crown gall tumor markers in transformants obtained after in vitro Agrobacterium tumefaciens - induced transformation of cell wall regenerating protoplasts derived from Nicotiana tabacum. Proc Natl Acad Sci 78: 4344-4348 Zambryski P, Joos H, Genetello C, van Montagu M, Schell J (1983) Ti-plasmid vector for the introduction of DNA into plant cells without altering their normal regeneration capacity, EMBO J 12: 2143-2150.

Reiss B, Sprengel R, Will H and Schaller H (1984) A new sensitive method for qualitative and quantitative assay of neomycin phosphotransferase in crude cell tracts, GENE 1081: 211-217

Schreier P, Seftor E, Schell J and Bohnert H (1985) The use of nuclear-encoded sequences to direct the light-regulated synthesis and transport of a foreign protein into plant chloroplasts, EMBO J Vol. 4, No. 1: 25-32

Weiterhin können die folgenden veröffentlichten Patentanmeldungen aufgeführt werden:

EP-A 116 718

EP-A 159 418

EP-A 120 515

EP-A 120 516

EP-A 172 112

EP-A 140 556

EP-A 174 166

EP-A 122 791

EP-A 126 546
EP-A 164 597
EP-A 175 966
WO 84/02913
WO 84/02919
WO 84/02920
WO 83/01176


Erläuterungen zu Fig. 1

Fig. 1    stellt das Plasmid p8.1.1 dar. Die Bibenzylsynthase-cDNA liegt auf dem ca. 1,6 kb großen Mlu
I Fragment.
In Fig. 1 bedeuten:
E : EcoRl    S: Sal I
B : Bam HI    P: Pst I
H : Hind III    N: Not I
PL: Polylinker aus dem Plasmid pSport 1

**Patentansprüche**

1.  Bibenzylsynthase-Gene.

2.  Bibenzylsynthase-Gene, welche dadurch gekennzeichnet sind, daß sie mit der im Plasmid p8.1.1 enthaltenen Bibenzylsynthase-cDNA Sequenz oder ihren Teilen bzw. mit der cDNA-Sequenz gemäß SEQ ID NO:1 oder ihren Teilen hybridisieren und für Bibenzylsynthase codieren.

3.  Bibenzylsynthase-Gene gemaß den Ansprüchen 1 und 2, erhältlich aus Orchideen.

4.  Bibenzylsynthase-Gene gemäß den Ansprüchen 1 bis 3, erhältlich aus Phalaenopsis spp., Bletilla striata oder Epipactis palustris.

5.  Regulatorisch wirkender Teil, insbesondere Promotor, der Bibenzylsynthase-Gene gemäß den Ansprüchen 1 bis 4.

6.  Struktur-Gene der Bibenzylsynthase-Gene gemäß den Ansprüchen 1 bis 4.

7.  Rekombinante prokaryontische oder eukaryontische DNA, welche ein oder mehrere Bibenzylsynthase-Gene oder ihre Teile gemäß den Ansprüchen 1 bis 6 als "fremde" DNA oder als "zusätzliche" DNA enthält.

8.  Rekombinante DNA gemäß Anspruch 7, welche in Pflanzenzellen (einschließlich Protoplasten) oder Pflanzen (einschließlich Pflanzenteilen und Samen) enthalten ist.

9.  Vektoren, welche ein oder mehrere Bibenzylsynthase-Gene oder ihre Teile gemäß den Ansprüchen 1 bis 8 und/oder rekombinante DNA gemäß Anspruch 7 enthalten.

10. Vektor-Plasmid P8.1.1.

11. Transformierte Mikroorganismen, welche ein oder mehrere Bibenzylsynthase-Gene oder ihre Teile gemäß den Ansprüchen 1 bis 6 und/oder rekombinante DNA gemäß Anspruch 7 oder Vektoren gemäß den Ansprüchen 9 und 10 enthalten.

12. Escherichia coli Stamm E. coli p8.1.1 sowie seine Mutanten.

13. Verwendung der Bibenzylsynthase-Gene und/oder ihrer Teile und/oder der rekombinanten DNA und/oder der Vektoren und/oder der transformierten Mikroorganismen gemäß den Ansprüchen 1 bis 12 zur Transformation von Pflanzenzellen (einschließlich Protoplasten) und Pflanzen (einschließlich Pflanzenteilen und Samen).

14. Transgene Pflanzenzellen (einschließlich Protoplasten) und Pflanzen (einschließlich Pflanzenteilen und Samen), welche ein oder mehrere Bibenzylsynthase-Gene und/oder deren Teile und/oder die rekombinante DNA gemäß den Ansprüchen 1 bis 7 als "fremde" oder "zusätzliche" DNA enthalten.

15. Verfahren zur Herstellung transgener Pflanzenzellen (einschließlich Protoplasten) und Pflanzen (einschließlich Pflanzenteile und Samen) mit erhöhter Resistenz gegen Schädlinge, dadurch gekennzeichnet, daß man
    (a) ein oder mehrere Bibenzylsynthase-Gene oder ihre Teile und/oder die rekombinante DNA gemäß den Ansprüchen 1 bis 7 in den Genom von Pflanzenzellen (einschließlich Protoplasten) einsetzt und

23

gegebenenfalls

(b) aus den transgenen Pflanzenzellen (einschließlich Protoplasten) vollständige transformierte Pflanzen regeneriert und gegebenenfalls vermehrt und gegebenenfalls

(c) von den so erhaltenen transgenen Pflanzen der Elterngeneration oder weiterer daraus gewonnener Generationen die gewünschten Pflanzenteile (einschließlich Samen) gewinnt.

16. Verwendung der transgenen Pflanzenzellen (einschließlich Protoplasten) und Pflanzen (einschließlich Pflanzenteilen und Samen) gemäß Anspruch 14 zur Erzeugung von Vermehrungsmaterial sowie zur Erzeugung neuer Pflanzen, die die Bibenzylsynthase-Gene oder ihre Teile gemäß den Ansprüchen 1 bis 6 oder die rekombinante DNA gemäß Anspruch 7 enthalten und deren Vermehrungsmaterial.

17. Vermehrungsmaterial, erhältlich durch die Vermehrung der transgenen Pflanzenzellen und Pflanzen gemäß Anspruch 14.

18. Verwendung von DNA-Sequenzen, welche ganz oder teilweise der cDNA entsprechen, die auf dem Plasmid P8.1.1 enthalten ist, bzw. in SEQ ID NO:1 aufgeführt ist, zur Isolierung von Bibenzylsynthase-Genen aus Pflanzen sowie bei der Erzeugung von transgenen Pflanzenzellen (einschließlich Protoplasten) und Pflanzen (einschließlich Pflanzenteilen und Samen).

19. Bibenzylsynthase, welche durch die erfindungsgemäßen Bibenzylsynthase-Gene codiert wird.

20. Bibenzylsynthase, welche durch das auf dem Plasmid p8.1.1 enthaltene Bibenzylsynthase-Strukturgen codiert wird.

21. Protein gemäß SEQ ID NO:2.

22. Verwendung der Bibenzylsynthase bzw. des Proteins gemäß den Ansprüchen 19 bis 21 für den Nachweis von Bibenzylsynthase.

FIG.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | PHYTOCHEMISTRY, Bd.30, Nr.2, 1991 Seiten 457 - 460 GEHLERT, R., ET AL. 'Induced formation of dihydrophenanthrenes and bibenzyl synthase upon destruction of orchid mycorrhiza' * das ganze Dokument * --- | 19,20 | C12N15/52 C12N15/82 C12N9/00 C12N5/10 C12N1/21 A01H5/00 C12Q1/00 |
| P,X | PHYTOCHEMISTRY, Bd.35, Nr.1, 1994 Seiten 63 - 66 REINECKE, T., ET AL. 'Charcterization of bibenzyl synthase catalysing the biosynthesis of phytoalexins of orchids' * das ganze Dokument * --- | 19,20 | |
| D,A | EP-A-0 464 461 (BAYER) 8. Januar 1992 * das ganze Dokument * --- | 1-22 | |
| A | NATURE, Bd.361, 14. Januar 1993 Seiten 153 - 156 HAIN, R., ET AL. 'Disease resistance results from foreign phytoalexin expression in a novel plant' * das ganze Dokument * --- | 1-18 | RECHERCHIERTE SACHGEBIETE (Int.Cl.6) C12N A01H C12Q |
| D,A | EP-A-0 309 862 (BAYER) 5. April 1989 * das ganze Dokument * --- | 1-22 | |
| A | PLANT MOLECULAR BIOLOGY, Bd.15, 1990 Seiten 325 - 335 HAIN, R., ET AL. 'Expression of a stilbene synthase gene in Nicotiana tabacum results in synthesis of the phytoalexin reservatrol' * das ganze Dokument * --- | 1-18 | |

-/--

Der vorliegende Recherchenbericht wurde für alle Patentansprüche erstellt

| Recherchenort | Abschlußdatum der Recherche | Prüfer |
|---|---|---|
| DEN HAAG | 30. Januar 1995 | Maddox, A |

EPO FORM 1503 03.82 (P04C03)

Europäisches
Patentamt

**EUROPÄISCHER RECHERCHENBERICHT**

Nummer der Anmeldung

EP 94 11 5471

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 533 010 (BAYER) 24. März 1993 * das ganze Dokument * ----- | 1-22 | |
| | | | **RECHERCHIERTE SACHGEBIETE (Int.Cl.6)** |

Der vorliegende Recherchenbericht wurde für alle Patentansprüche erstellt

| Recherchenort | Abschlußdatum der Recherche | Prüfer |
|---|---|---|
| DEN HAAG | 30. Januar 1995 | Maddox, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.......................................................................
& : Mitglied der gleichen Patentfamilie, übereinstimmendes
    Dokument

EPO FORM 1503 03.82 (P04C03)

SEQUENZPROTOKOLL

(1) ALGEMEINE INFORMATION:

    (i) ANMELDER:
        (A) NAME: Bayer AG
        (B) STRASSE: Bayerwerk
        (C) ORT: Leverkusen
        (E) LAND: Deutschland
        (F) POSTLEITZAHL: D-51368
        (G) TELEPHON: 0214/30 66400
        (H) TELEFAX: 0214/30 3482
        (I) TELEX: 85 101-265 by d

    (ii) ANMELDETITEL: Bibenzylsynthase-Gene

   (iii) ANZAHL DER SEQUENZEN: 2

   (iv) COMPUTER-LESBARE FORM:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.25 (EPA)

(2) INFORMATION ZU SEQ ID NO: 1:

    (i) SEQUENZ CHARAKTERISTIKA:
        (A) LÄNGE: 453 Basenpaare
        (B) ART: Nukleinsäure
        (C) STRANGFORM: Einzel
        (D) TOPOLOGIE: linear

    (ii) ART DES MOLEKÜLS: DNS (genomisch)

   (iii) HYPOTHETISCH: NEIN

   (iii) ANTISENSE: NEIN

    (ix) MERKMALE:
        (A) NAME/SCHLÜSSEL: CDS
        (B) LAGE: complement (1..453)

    (ix) MERKMALE:
        (A) NAME/SCHLÜSSEL: CDS
        (B) LAGE: 1..453

    (xi) SEQUENZBESCHREIBUNG: SEQ ID NO: 1:

```
ATG CCG AGC CTT GAA TCC ATC AAG AAG GCC CCA AGA GCC GAC GGC TTC      48
Met Pro Ser Leu Glu Ser Ile Lys Lys Ala Pro Arg Ala Asp Gly Phe
 1               5                  10                  15

GCC TCC ATT TTG GCC ATC GGG AGG GCG AAC CCA GAC AAT ATT ATT GAA      96
Ala Ser Ile Leu Ala Ile Gly Arg Ala Asn Pro Asp Asn Ile Ile Glu
            20                  25                  30

CAG AGC GCC TAC CCA GAC TTC TAC TTT CGT GTC ACC AAT AGC GAG CAC     144
Gln Ser Ala Tyr Pro Asp Phe Tyr Phe Arg Val Thr Asn Ser Glu His
            35                  40                  45
```

21

```
TTG GTC GAC CTC AAA AAG AAA TTT CAA CGC ATC TGT GAG AAG ACG GCA        192
Leu Val Asp Leu Lys Lys Lys Phe Gln Arg Ile Cys Glu Lys Thr Ala
    50              55                  60

ATC AGA AAG CGC CAC TTT GTC TGG AAC GAG GAG TTT CTG ACT GCA AAC        240
Ile Arg Lys Arg His Phe Val Trp Asn Glu Glu Phe Leu Thr Ala Asn
65              70                  75                  80

CCT TGC TTC AGC ACA TTC ATG GAC AAA TCT TTA AAC GTA AGG CAA GAG        288
Pro Cys Phe Ser Thr Phe Met Asp Lys Ser Leu Asn Val Arg Gln Glu
                85                  90                  95

GTT GCT ATA AGC GAG ATA CCA AAA CTG GGC GCG AAG GCG GCC ACC AAG        336
Val Ala Ile Ser Glu Ile Pro Lys Leu Gly Ala Lys Ala Ala Thr Lys
            100                 105                 110

GCT ATC GAG GAC TGG GGG CAG CCT AAA TCG CGT ATA ACT CAC CTA ATC        384
Ala Ile Glu Asp Trp Gly Gln Pro Lys Ser Arg Ile Thr His Leu Ile
        115                 120                 125

TTC TGC ACC ACG AGC GGC ATG GAC TTA CCT GGT GCT GAT TAC CAG CTC        432
Phe Cys Thr Thr Ser Gly Met Asp Leu Pro Gly Ala Asp Tyr Gln Leu
    130                 135                 140

ACC CAA ATC CCA ATG TTG AGC                                            453
Thr Gln Ile Pro Met Leu Ser
145                 150
```

(2) INFORMATION ZU SEQ ID NO: 2:

    (i) SEQUENZ CHARAKTERISTIKA:
      (A) LÄNGE: 151 Aminosäuren
      (B) ART: Aminosäure
      (D) TOPOLOGIE: linear

    (ii) ART DES MOLEKÜLS: Protein

    (xi) SEQUENZBESCHREIBUNG: SEQ ID NO: 2:

```
Met Pro Ser Leu Glu Ser Ile Lys Lys Ala Pro Arg Ala Asp Gly Phe
1               5               10                  15

Ala Ser Ile Leu Ala Ile Gly Arg Ala Asn Pro Asp Asn Ile Ile Glu
            20                  25                  30

Gln Ser Ala Tyr Pro Asp Phe Tyr Phe Arg Val Thr Asn Ser Glu His
        35                  40                  45

Leu Val Asp Leu Lys Lys Lys Phe Gln Arg Ile Cys Glu Lys Thr Ala
    50              55                  60

Ile Arg Lys Arg His Phe Val Trp Asn Glu Glu Phe Leu Thr Ala Asn
65              70                  75                  80

Pro Cys Phe Ser Thr Phe Met Asp Lys Ser Leu Asn Val Arg Gln Glu
                85                  90                  95

Val Ala Ile Ser Glu Ile Pro Lys Leu Gly Ala Lys Ala Ala Thr Lys
            100                 105                 110

Ala Ile Glu Asp Trp Gly Gln Pro Lys Ser Arg Ile Thr His Leu Ile
        115                 120                 125
```

EP 0 648 839 A1

```
Phe Cys Thr Thr Ser Gly Met Asp Leu Pro Gly Ala Asp Tyr Gln Leu
    130                 135                 140

Thr Gln Ile Pro Met Leu Ser
145                 150
```